# DEMANDE DE BREVET EUROPEEN

(11) **EP 4 579 670 A1**
(43) Date de publication de la demande: **02.07.2025**
(21) Numéro de dépôt: 24223297.3
(22) Date de dépôt: 26.12.2024
(51) Int. Cl.: G16B 40/10, C12Q 1/04, C12Q 1/18, G01N 21/35, G01N 21/3577, G01N 21/65, G01N 33/68

(54) **PROCÉDÉ DE DÉTERMINATION D'UNE RÉSISTANCE D'UN MICROORGANISME À L ÉGARD D'UN ANTIMICROBIEN**

(30) Priorité: 26.12.2023 FR 2315333
(71) Demandeur: Commissariat à l'Energie Atomique et aux Energies Alternatives, 75015 Paris (FR)
(72) Inventeur: PISSOT, STÉPHAN, 38054 Grenoble cedex 09 (FR); DUPOY, MATHIEU, 38054 Grenoble cedex 09 (FR); MARCOUX, PIERRE, 38054 Grenoble cedex 09 (FR)
(74) Mandataire: INNOV-GROUP

(57) **Abrégé**

Procédé de d'apprentissage d'un algorithme de caractérisation, configuré pour caractériser un métabolisme d'au moins une souche de microorganisme, le procédé comportant les étapes suivantes:
- a) la répartition de différents microorganismes de ladite souche dans différents échantillons d'apprentissage, les échantillons d'apprentissage comportant un milieu nutritif auquel a été ajouté un marqueur métabolique respectivement selon différentes concentrations, le marqueur métabolique étant destiné à être métabolisé par chaque microorganisme, au moins un échantillon étant tel qu'il ne comporte pas de marqueur métabolique, ou selon une concentration considérée comme négligeable ;
- b) l'acquisition de spectres d'apprentissage de microorganismes de chaque échantillon d'apprentissage ;
- c) à partir des spectres d'apprentissage acquis dans chaque échantillon d'apprentissage, le paramétrage de l'algorithme de caractérisation, de façon à caractériser, à partir de chaque spectre d'apprentissage, un métabolisme du microorganisme. Figure 4C

## Description

### DOMAINE TECHNIQUE

Le domaine technique de l'invention est la détermination d'une résistance d'un microorganisme à un traitement, en particulier un traitement par antibiotique ou un antifongique.

### ART ANTERIEUR

L'antibiorésistance désigne la capacité d'une bactérie à se développer en présence d'une certaine concentration d'antibiotique. Il s'agit d'un problème de santé publique, car certaines bactéries ont déjà acquis une résistance à l'égard de certains antibiotiques. On considère aujourd'hui que quelques bactéries ont acquis une résistance à l'égard d'un grand nombre d'antibiotiques.

La sensibilité d'une bactérie à l'égard d'un antibiotique désigne la capacité de la bactérie à se développer en présence d'un agent antibiotique. Différentes méthodes existent, pour déterminer la sensibilité d'une bactérie à un antibiotique : Il peut par exemple s'agir d'une mise en culture de bactéries soumises à différentes concentrations d'antibiotiques. Cependant, un tel procédé est long à mettre en oeuvre.

Des méthodes génomiques ont été mises en oeuvre, qui visent à cibler des séquences de gènes considérées comme marqueurs d'antibiorésistance. Mais il s'agit de méthodes onéreuses, qui sont encore peu usuelles en pratique courante.

La publication Sharaha U, « Using infrared spectroscopy and multivariate analysis to detect antibiotics'resistant Escherichia coli Bacteria", Anal. Chem 2017, 89, 8782 - 8790 décrit une méthode de détection de l'antibiorésistance de bactéries en mettant en oeuvre la spectrométrie infrarouge, usuellement désignée par l'acronyme anglais (FTIR) Fourier Transformed Infrared Spectroscopy. La spectrométrie infrarouge est une technique de mesure basée sur la transmission d'un rayonnement infrarouge à travers ou en surface d'un échantillon biologique. Elle permet via la détection des vibrations caractéristiques des liaisons chimiques, d'effectuer une topographie des fonctions chimiques présentes dans l'échantillon. L'échantillon est illuminé par une source de lumière émettant dans l'infra-rouge selon une plage de longueurs d'onde généralement comprises entre (25 à 2,5 micromètres) ce qui équivaut à un nombre d'onde compris entre de 4000 à 400 cm⁻¹. Lorsque la longueur d'onde correspond à l'énergie de vibration ou d'absorption des molécules présentent dans l'échantillon, une partie de la lumière est absorbée. Cela se traduit par la détection de pics d'absorption par le photodétecteur.

Dans cette publication précitée, on décrit une méthode de détection d'une antibiorésistance basée sur une classification de spectres d'absorption infra-rouge acquis par FTIR. La classification est effectuée par un opérateur de classification binaire de type SVM (Support Vector Machine - Machine à support de vecteurs). L'opérateur est préalablement entraîné en utilisant des bactéries, en l'occurrence *E. Coli,* ayant préalablement fait un test d'antibiorésistance. Ainsi, la mise en oeuvre de la méthode suppose un apprentissage basé sur des bactéries dont l'antibiorésistance a été préalablement caractérisée.

Des méthodes analogues ont été décrites dans WO2022208104 (appliquée à la spectroscopie Raman), ou EA033790B1 (spectroscopie d'absorption infra-rouge), EP2845011 (spectrométrie de masse), US9927352B2 (FTIR), FR3108983 (imagerie infra-rouge), US2023028710 (spectroscopie Raman) ainsi que dans les publications :
- Stôckel « The application of Raman spectroscopy for the détection and identification of microorganisms : Raman spectroscopy for microorganism détection and identification », Journal of Raman Spectroscipy, vol. 47, n° 1, où l'on décrit une identification, par spectrométrie Raman, de l'interaction d'antibiotiques avec certaines bactéries ;
- Tang Wenli et al "MMINP : A computational framework of microbe-metabolite interactions-based metabolic profiles predictor based on the O2 PLS algorithm", Gut Microbes vol. 15, n°1, june 2023;
- Ralbovsky Nicole M. et al "Towards development of a novel universal medical diagnostic method : Raman spectroscopy and machine learning" Chemical Society reviews, Vol. 49, n°20, october 2020 ;
- Athamneh et al "Phenotypic profiling of antibiotic response signatures in Escherichia Coli using Raman Spectroscopy", Antimicrobial agents and chemotherapy, vol. 58, n°3, December 2013.

Dans CA3236928, on effectue une spectroscopie Raman pour analyser un milieu de culture, de façon à former des profils métaboliques de milieux de culture : il s'agit ici d'examiner les conséquences, sur le milieu de culture, et non sur le bactéries elles mêmes, de l'exposition de bactéries à un antibiotique

Dans la publication Zwielly A. « Discrimination between drug-resistant and non-resistant human melanoma cell lines by FTIR spectroscopy", Analyst, 2009, 134, 595-300, les auteurs décrivent le recours à la FTIR pour distinguer des cellules de mélanomes résistantes ou non à la cisplatine, qui est un principe actif utilisé en chimiothérapie. Plus précisément, ils mettent en évidence des différences spectrales entre des cellules résistantes et non résistances à la cisplatine.

Dans les documents pré-cités, une phase d'apprentissage est nécessaire, au cours de laquelle des microorganismes connus sont placés au contact d'agents antibiotiques connus. Cela suppose la formation de bases de données de mesures spécifiques à des couples microorganismes/antibiotiques.

Les inventeurs proposent une méthode différente, permettant d'évaluer, de préférence de façon non destructive, la résistance d'un microorganisme à l'égard d'un traitement de type antibiotique ou antifongique.

### EXPOSE DE L'INVENTION

Un premier objet de l'invention est un procédé de d'apprentissage d'un algorithme de caractérisation, configuré pour caractériser un métabolisme d'au moins une souche de microorganisme, le procédé comportant les étapes suivantes:
- a) la répartition de différents microorganismes de ladite souche dans différents échantillons d'apprentissage, les échantillons d'apprentissage comportant un milieu nutritif auquel a été ajouté un marqueur métabolique respectivement selon différentes concentrations, le marqueur métabolique étant destiné à être métabolisé par chaque microorganisme, au moins un échantillon étant tel qu'il ne comporte pas de marqueur métabolique, ou selon une concentration considérée comme négligeable ;
- b) l'acquisition de spectres d'apprentissage de microorganismes de chaque échantillon d'apprentissage ;
- c) à partir des spectres d'apprentissage acquis dans chaque échantillon d'apprentissage, le paramétrage de l'algorithme de caractérisation, de façon à caractériser, à partir de chaque spectre d'apprentissage, un métabolisme du microorganisme.

Selon un mode de réalisation :
- dans l'étape a), de microorganismes de différentes souches sont respectivement disposés dans différents échantillons d'apprentissage;
- l'étape b) comporte une acquisition de spectres d'apprentissage de microorganismes dans chaque échantillon d'apprentissage.

L'algorithme de caractérisation peut être configuré pour classer le métabolisme dans une classe choisie parmi :
- une réduction du métabolisme ;;
- un métabolisme normal.

Dans l'étape a), les microorganismes peuvent être disposées respectivement dans des milieux nutritifs identiques, les échantillons d'apprentissage différant les uns des autres par la concentration de marqueur métabolique ajoutée.

Le marqueur métabolique peut être un marqueur isotopique ou un marqueur chromogène. Selon une possibilité, dans l'étape b), les microorganismes de chaque échantillon d'apprentissage sont interposés entre une source de lumière infrarouge et un photodétecteur, chaque spectre d'apprentissage étant un spectre d'absorption ou de transmission dans l'infrarouge.

Selon une possibilité dans l'étape b), les microorganismes de chaque échantillon d'apprentissage sont disposés sur un support et exposées à un faisceau laser, chaque spectre d'apprentissage étant un spectre de masse.

Un deuxième objet de l'invention est un procédé de détermination d'une résistance d'un microorganisme à l'égard d'un agent antibiotique ou fongicide, le microorganisme étant disposé dans un échantillon d'analyse comportant le marqueur métabolique utilisé dans l'étape a) d'un procédé d'apprentissage selon le premier objet de l'invention, le procédé comportant les étapes :
- i) mise en contact du microorganisme avec l'agent antibiotique ou fongicide ;
- ii) acquisition d'un spectre d'analyse du microorganisme, le spectre d'analyse étant du même type que les spectres d'apprentissage acquis dans chaque étape b) du procédé d'apprentissage ;
- iii) application d'un algorithme de caractérisation tel que défini dans l'étape c) du procédé d'apprentissage, au spectre résultant de l'étape ii), de façon à caractériser le métabolisme du microorganisme ;
- iv) détermination de la résistance du microorganisme à l'égard de l'agent antibiotique ou fongicide en fonction de la caractérisation effectuée lors de l'étape iii).

Par spectre d'analyse de même type, on entend un spectre acquis par la même modalité d'analyse.

Selon une possibilité,
- on dispose le microorganisme dans différents échantillons d'analyse, comportant respectivement différentes concentrations de l'antibiotique ;
- les étapes i) à iii) sont mises en oeuvre avec chaque échantillon d'analyse ;
- l'étape iv) comporte une détermination d'une concentration d'antibiotique ou de fongicide au-delà de laquelle le microorganisme n'est plus résistant.

Selon une possibilité,
- le procédé d'apprentissage est mis en oeuvre avec des microorganismes d'apprentissage ;
- le procédé d'analyse est mis en oeuvre avec un microorganisme différent des microorganismes d'apprentissage.

Un troisième objet de l'invention est un dispositif, comportant un spectromètre, configuré pour acquérir un spectre d'analyse d'un microorganisme, le dispositif comportant une unité de traitement programmée pour mettre en oeuvre un procédé selon le deuxième objet de l'invention.

Le spectromètre peut être :
- un spectromètre infra-rouge ;
- ou un spectromètre de masse ;
- ou un spectromètre Raman.

L'invention sera mieux comprise à la lecture de l'exposé des exemples de réalisation présentés, dans la suite de la description, en lien avec les figures listées ci-dessous.

### FIGURES

La figure 1 schématise les principaux composants d'un dispositif configuré pour mettre en oeuvre l'invention.
La figure 2 montre les étapes principales d'un procédé d'apprentissage et d'un procédé d'analyse.
La figure 3 illustre des étapes du procédé d'analyse.
La figure 4A montre un spectre d'absorption d'un échantillon.
La figure 4B montre un vecteur de pondération résultant d'une analyse discriminante linéaire.
La figure 4C représente une caractérisation du métabolisme (axe des ordonnées), déterminés par un algorithme de caractérisation, en fonction d'une concentration d'antibiotique (axe des abscisses). Sur la figure 4C, on a caractérisé le métabolisme d'une bactérie ayant été utilisée lors de l'apprentissage.
La figure 5 représente des états de métabolisme (axe des ordonnées), déterminés par un algorithme de caractérisation, en fonction d'une concentration d'antibiotique (axe des abscisses). Sur la figure 5, on a caractérisé le métabolisme d'une bactérie n'ayant pas été utilisée lors de l'apprentissage.
La figure 6 représente une détermination de bandes spectrales pouvant être utilisées pour une mise en oeuvre de l'invention.

### EXPOSE DE MODES DE REALISATION PARTICULIERS

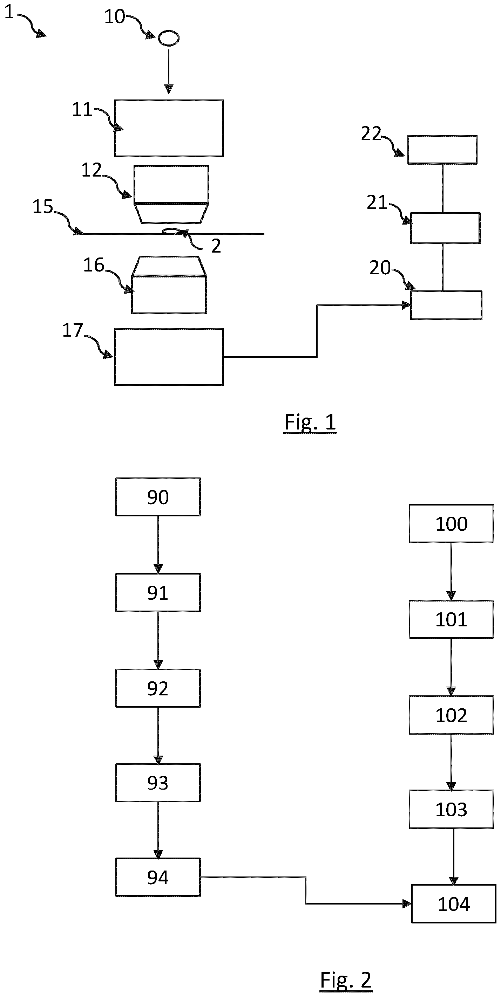

La figure 1 représente un exemple de dispositif d'analyse 1 permettant une mise en oeuvre de l'invention. Dans cet exemple, le dispositif d'analyse est un spectromètre de type FTIR (Fourier Transform -Infra Red), ce qui signifie infra-rouge à transformée de Fourier. Ce type de dispositif, connu de l'homme du métier, permet d'acquérir un spectre d'analyse représentatif de la transmission de la lumière par l'échantillon. Un tel spectre permet une identification de raies d'absorption, qui constituent une signature de la composition de l'échantillon.

Le dispositif 1 comporte une source de lumière 10 configurée pour émettre un faisceau lumineux, dit faisceau lumineux incident, vers un échantillon 2. Le faisceau lumineux est émis dans une bande spectrale infra-rouge, typiquement entre 2.5 µm (soit un nombre d'onde de 4000 cm⁻¹) et 16 µm (soit un nombre d'onde de 600 cm⁻¹).Le dispositif 1 comporte également un interféromètre 11. Le faisceau issu de l'interféromètre est focalisé sur l'échantillon 2 par un objectif amont 12. Le faisceau transmis par l'échantillon 2 est collecté par un objectif aval 16, ce dernier étant optiquement couplé à un photodétecteur 17.

Le photodétecteur 17 est relié à une unité de traitement 20, programmée pour établir un spectre correspondant à une intensité de la lumière transmise par l'échantillon en fonction de la longueur d'onde. Le spectre fait apparaître des raies d'absorption, qui correspondent spécifiquement à des liaisons présentes dans l'échantillon. L'unité de traitement 20 comporte un microprocesseur programmé pour former le spectre à partir des mesures résultant du photodétecteur. L'unité de traitement 20 est reliée à une mémoire 21, dans laquelle les instructions de traitement des mesures sont codées. Le terme unité de traitement est à considérer au sens large. Il peut s'agir d'un ou plusieurs microprocesseurs, reliés physiquement au photodétecteur 17 ou par une liaison sans fil.

Dans cet exemple, l'échantillon 2 est une goutte comportant des microorganismes que l'on souhaite analyser. La goutte est déposée sur une lame transparente 15 faisant office de support d'échantillon. La lame 15 est transparente vis-à-vis du faisceau incident.

Par microorganismes, on entend des bactéries, mais également des champignons, des microalgues ou des procaryotes. Dans l'exemple qui suit le microorganisme est une bactérie.

Dans l'art antérieur, on a décrit un procédé basé sur un apprentissage au cours duquel des bactéries sont disposées dans un milieu comportant un antibiotique. L'objectif est de déterminer une signature spectrale de l'action de l'antibiotique. Cela suppose l'élaboration d'une base de données comportant des spectres représentatifs d'une dégradation de la bactérie par l'antibiotique. On peut présumer que la signature spectrale peut varier en fonction de la souche bactérienne ainsi qu'en fonction de l'antibiotique utilisé. Il en résulte la nécessité de disposer de bases de données spécifiques à des couples souche bactérienne / antibiotique.

L'approche suivie par les inventeurs est différente. Il s'agit d'obtenir une signature spectrale représentative d'une modification du métabolisme d'une bactérie quelconque, et cela quel que soit l'antibiotique mis en oeuvre.

Par métabolisme, on entend l'ensemble des réactions enzymatiques ayant lieu à l'intérieur de la cellule. Pour survivre et se reproduire, les bactéries transforment en permanence des molécules issues de leur environnement et produisent des biomolécules plus complexes. Par conséquent, quantifier l'effet d'un antibiotique sur ces réactions permet d'obtenir une information sur la sensibilité d'une bactérie à une concentration donnée d'antibiotique, sans avoir à attendre sa croissance.

Afin de pouvoir suivre l'évolution du métabolisme, les bactéries sont disposées dans un milieu nutritif comportant un marqueur métabolique. Le marqueur métabolique peut être marqueur isotopique (par exemple deutérium ²H, usuellement noté D, ou ¹³C), ou une molécule présentant une liaison absorbant la lumière infra-rouge dans une longueur d'onde prédéterminée. Lors de l'utilisation d'un marqueur isotopique, D peut être incorporé par exemple à de l'eau (eau lourde) et ¹³C peut être incorporé à du glucose. La fonction du marqueur métabolique est de pouvoir suivre, par une méthode d'analyse, et en particulier une méthode d'analyse spectrale, une évolution du métabolisme de la bactérie. De préférence, la méthode d'analyse spectrale est non destructive. C'est pourquoi la méthode d'analyse privilégiée est la spectrométrie FTIR. L'invention peut s'appliquer à d'autres méthodes d'analyse spectrales, comme décrit par la suite.

D'une façon générale, la méthode est essentiellement basée sur l'utilisation d'un classifieur, qui, à partir d'un spectre d'analyse, par exemple un spectre FTIR, permet d'évaluer le métabolisme d'une bactérie. Plus précisément, le classifieur est programmé pour classer une bactérie, sur la base du spectre d'analyse, en une classe représentative de son métabolisme. La caractérisation peut permettre une classification est effectuée entre au moins 2 classes, correspondant à la présence d'un métabolisme et à l'absence ou une réduction de métabolisme, auquel cas la bactérie est considérée comme morte ou dégradée. De préférence, la classification est effectuée entre différentes classes, chaque classe correspondant à un certain niveau de métabolisme.

La figure 2 schématise les principales étapes d'une phase d'apprentissage, permettant de paramétrer le classifieur. Les étapes sont référencées 90, 91, 92, 93 et 94.

Etape 90 : les bactéries d'une même souche, ou de différentes souches, sont réparties de façon à constituer différents échantillons d'apprentissage. Chaque échantillon d'apprentissage comporte un même milieu nutritif. Certains échantillons d'apprentissage comportent le marqueur métabolique, la concentration de ce dernier étant connue et variable entre les différents échantillons. Certains échantillons d'apprentissage ne comportent pas de marqueur métabolique, ou à une concentration considérée comme négligeable.

Préalablement à l'étape 90, une préculture peut être effectuée, de façon à synchroniser les états métaboliques des bactéries qui seront réparties dans les différents échantillons d'apprentissage. La préculture peut durer plusieurs heures, par exemple une nuit. Les différentes bactéries étant disposées, durant un période de temps assez longue, dans un même milieu nutritif, leurs métabolismes respectifs peuvent être considérés comme comparables à l'issue de la préculture : on parle de synchronisation des métabolismes.

De préférence, on utilise une grande variété de souches bactériennes, par exemple des souches gram positif, gram négatif, coques/bacilles.

Etape 91 : incubation. Au cours de cette étape, chaque échantillon d'apprentissage est ensuite incubé pendant une durée de quelques heures, par exemple deux heures.

Etape 92 : lavage : les échantillons d'apprentissage sont lavés c'est-à-dire centrifugés et re-suspendus dans l'eau. Cela permet d'éliminer, dans chaque échantillon d'apprentissage, des résidus de marqueurs métaboliques non consommés par les bactéries.

Etape 93 : acquisition de spectres d'analyse. Des spectres d'analyse des bactéries, résultant de chaque échantillon lavé, sont acquis. Dans cet exemple, les spectres d'analyse sont des spectres FTIR.

Suite à l'étape 93, on dispose d'une grande quantité de spectres d'apprentissage, provenant de bactéries marquées et non marquées. Afin de pouvoir caractériser le marquage, il est préférable, mais non nécessaire, que les étapes 90 à 93 soient mises en oeuvre en utilisant une diversité de souches bactériennes. On dispose ainsi de spectres d'analyse respectivement représentatives de bactéries marquées, et éventuellement avec différents niveaux de marquage, et des bactéries non marquées. Le fait de recourir à une diversité de souches bactériennes permet une meilleure caractérisation du marquage par l'algorithme de caractérisation décrit par la suite. Il s'agit d'obtenir une caractérisation qui soit la plus universelle possible, et non spécifique au métabolisme d'une souche particulière.

Etape 94 : paramétrage de l'algorithme de caractérisation. Au cours de cette étape, on effectue un apprentissage de l'algorithme de caractérisation, en utilisant les différents spectres d'analyse résultant de l'étape 93, sachant qu'à chaque spectre est associé un niveau de marquage connu : absence de marquage, présence de marquage, et éventuellement différents niveaux de marquage.

L'objectif de l'apprentissage est que de l'algorithme de caractérisation puisse, à partir d'un spectre, caractériser un niveau de marquage : absence de marquage et présence de marquage, et éventuellement présence de différents niveaux non nuls de marquage. Ainsi, chaque classe est représentative d'un niveau de marquage, deux classes différentes étant respectivement représentatives de deux niveaux de marquage différents. Au moins une classe est représentative d'un niveau de marquage nul, ou pouvant être considéré comme tel. Lors de la mise en oeuvre de la méthode sur des échantillons inconnus, le niveau de marquage peut être traduit en un niveau de métabolisme : plus le niveau de marquage est élevé, plus le métabolisme est considéré comme important.

L'algorithme de caractérisation peut être un algorithme de classification, par exemple de type analyse par discriminant linéaire (Linear Discriminant Analysis - LDA), réseau de neurones, ou une régression par moindre carrés partiels (PLS : Partial least square).

L'algorithme de caractérisation ainsi paramétré est destiné à être utilisé pour analyser le métabolisme d'une bactérie en présence d'un antibiotique et du marqueur utilisé lors de l'apprentissage, sur la base d'un spectre d'analyse. Une présence d'un métabolisme actif résulte d'une certaine résistance à l'antibiotique. Une absence de métabolisme, se traduisant par un niveau de marquage nul, traduit une sensibilité à l'antibiotique.

Contrairement à l'art antérieur :
- l'apprentissage de l'algorithme de caractérisation n'est pas effectué en présence d'un antibiotique ;
- l'apprentissage de l'algorithme de caractérisation est effectué préférentiellement en utilisant des souches bactériennes différentes. Il est préférable qu'une même souche bactérienne soit marquée et non marquée, c'est-à-dire disposée dans des échantillons d'apprentissage avec et sans marqueur métabolique.

L'algorithme de caractérisation ayant été paramétré, les étapes d'analyse d'une antibiorésistance peuvent être effectuées. Il s'agit d'analyser le métabolisme d'une bactérie en présence d'un antibiotique, et de préférence de différentes concentrations de l'antibiotique. L'objectif est de déterminer la concentration à laquelle la bactérie ne présente plus de métabolisme ou un métabolisme réduit.

Au cours d'une étape 100, les bactéries d'une même souche sont réparties dans différents échantillons d'analyse. Il s'agit de préférence de bactéries ayant été utilisées lors de l'apprentissage, cette condition n'étant pas nécessaire. Chaque échantillon d'analyse comporte un même milieu nutritif, de préférence similaire à celui utilisé lors de la phase d'apprentissage. Le milieu nutritif comporte le même marqueur métabolique que celui utilisé lors de la phase d'apprentissage. Les échantillons d'analyse comportent différentes concentrations d'un antibiotique.

Au cours d'une étape 101, chaque échantillon est maintenu en incubation, de préférence selon une durée identique ou comparable à la durée d'incubation de la phase d'apprentissage. Cf. étape 91.

Au cours d'une étape d'incubation 102, un prélèvement de chaque échantillon est effectué, puis lavé et disposé sur le support d'analyse 15.

Au cours d'une étape d'analyse 103, chaque prélèvement fait l'objet d'une analyse, de façon à acquérir un spectre d'analyse. On obtient ainsi au moins autant de spectres d'analyse que d'échantillons d'analyse. De préférence, plusieurs spectres d'analyse, typiquement plusieurs dizaines, sont effectués pour chaque échantillon, et éventuellement moyennés.

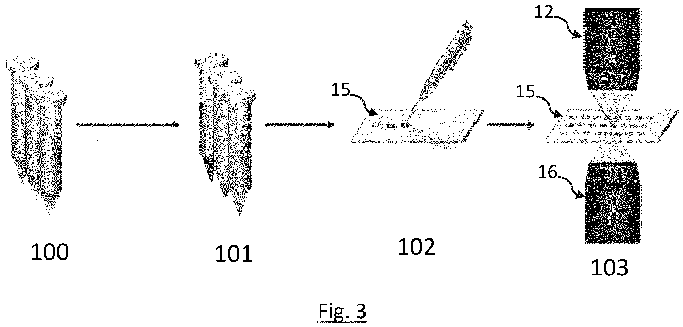

Les étapes 100 à 103 sont illustrées sur la figure 3.

Au cours d'une étape 104, l'algorithme de caractérisation, paramétré durant la phase d'apprentissage, est appliqué à chaque spectre d'analyse, de façon à caractériser le métabolisme de l'échantillon.. Les spectres d'analyse correspondant aux échantillons présentant une faible concentration d'antibiotique sont considérés comme représentatifs d'un métabolisme actif : cela traduit le fait que les bactéries métabolisent le milieu nutritif en présence de l'antibiotique. Les spectres d'analyse correspondant aux échantillons présentant une forte concentration d'antibiotique sont considérés comme représentatifs d'un métabolisme ralenti ou inactif : cela traduit le fait que les bactéries meurent et ne métabolisent le milieu nutritif en présence de l'antibiotique.

L'algorithme de caractérisation permet de distinguer entre un métabolisme normal, qui correspond à une absence de perturbation du métabolisme de la bactérie, et un métabolisme ralenti, qui correspond à un ralentissement du métabolisme de la bactérie sous l'effet de la concentration ajoutée d'antibiotique. Lors de l'apprentissage, les classes correspondant respectivement au métabolisme normal et au métabolisme ralenti correspondent à des échantillons d'apprentissage dans lesquels la concentration de marqueur métabolique est respectivement forte et faible. On comprend que la concentration de marqueur métabolique, i-e le niveau de marquage, est représentatif de l'activité du métabolisme.

La comparaison des caractérisations de chaque échantillon d'analyse permet de déterminer une concentration, , au-delà de laquelle la bactérie est considérée comme sensible à l'antibiotique.

Les inventeurs ont mis en oeuvre les phases d'apprentissage (étapes 90 à 94) et d'analyse (étapes 100 à 104).

Au cours de l'apprentissage, on a utilisé huit souches d'espèces de bactéries différentes pour constituer les échantillons d'apprentissage (*Escherichia. coli, Klebsiella. cerogenes, Citrobacter. freundii, Sophylococcus. epidermedis, Pseudomonas putida* et *Sophylococcus. lentus*).

Pour chacune de ces souches, une pré-culture d'une nuit a été effectuée afin de synchroniser les états métaboliques des bactéries, puis deux cultures de deux heures ont été effectuées à partir de celle-ci : une dans 2mL de milieu de Mueller-Hinton (le milieu nutritif de référence pour la réalisation des antibiogrammes) et 50% v/v d'eau lourde, et une dans le milieu de Mueller-Hinton seul, sans eau lourde.

Après deux heures d'incubation, les échantillons d'apprentissage ont été centrifugés et re-suspendus dans 30µL d'eau puis une goutte de 5µL a été déposée sur une lame de fluorure de calcium (CaF2, substance transparente aux infrarouges) faisant office de support d'analyse. La lame a été séchée par exposition à une atmosphère chaude et sèche.

Pour chaque échantillon d'apprentissage, 64 spectres ont été acquis à l'aide d'un microscope FTIR à un grandissement de 15x. Le nombre élevé de spectres a été déterminé pour s'abstraire de potentielles variations d'épaisseur ou de texture du dépôt. Pour chaque échantillon d'apprentissage, on a formé un spectre d'apprentissage à partir d'une moyenne des spectres acquis)

Les spectres d'apprentissage obtenus ont été organisés en deux classes : ceux qui correspondent aux échantillons « marqués » (milieu nutritif comportant de l'eau lourde) et ceux correspondant aux échantillons non marqués. Une régression des moindres carrés partiels (PLS) a été ensuite entrainée sur ce jeu de données. On obtient alors un modèle capable de quantifier le marquage à l'eau lourde d'un échantillon par son spectre FTIR.

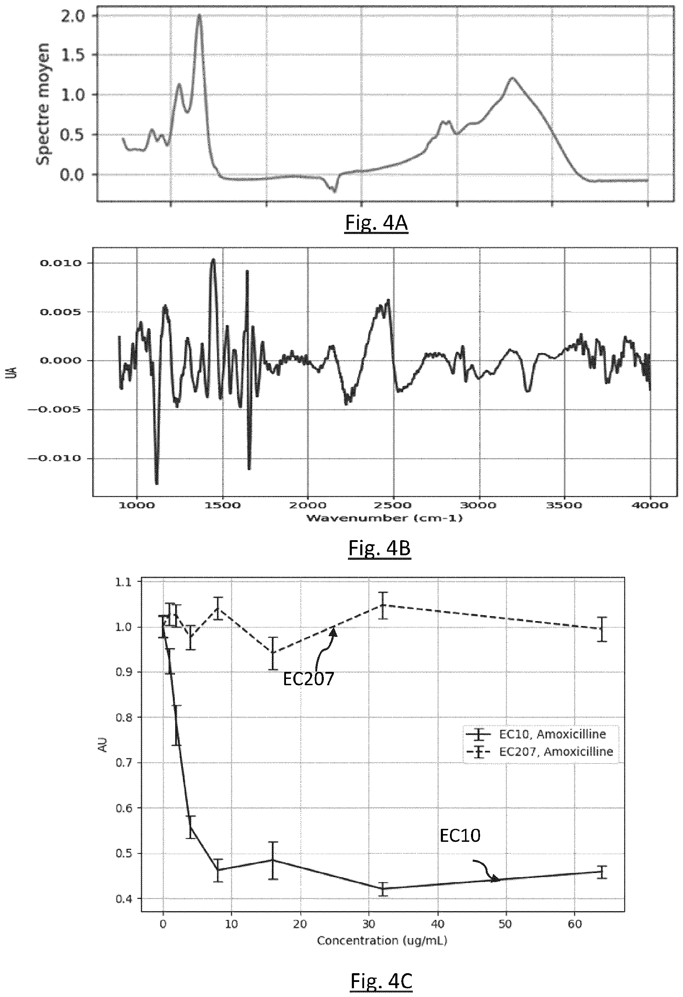

La figure 4A montre un exemple de spectre d'absorption d'un échantillon d'apprentissage, comportant un milieu nutritif de Mueller-Hinton marqué par du Deutérium. L'axe des abscisses correspond au nombre d'ondes (unité cm⁻¹).

La figure 4B montre un exemple de vecteur de pondération produit par analyse discriminante linéaire (LDA) des spectres de calibration. Ce vecteur représente les poids (axe des ordonnées) à donner à chaque nombre d'onde (axe des abscisses) pour obtenir un discriminant séparant les populations incubées en présence d'eau lourde de celles incubées en son absence. Dans le vecteur représenté sur la figure 4B, les nombres d'onde correspondant aux poids ayant les valeurs absolues les plus élevées sont considérés comme les plus discriminants entre deux classes.

Suite au paramétrage du classifieur LDA, ce dernier a été utilisé lors d'une phase d'analyse, sur des cultures d'*Escherichia coli.* Pour valider l'analyse, deux souches de référence ont été utilisées : la souche ATCC25922, notée ici « EC10 » est sensible à l'amoxicilline, présentant notamment une inhibition de sa croissance à partir d'une concentration de 4µg/mL d'antibiotique ; la souche ATCC35218 notée « EC207 » est quant à elle résistante à l'amoxicilline.

Une pré-culture d'une nuit a été effectuée pour synchroniser les états métaboliques des populations bactériennes. On a constitué des échantillons d'analyse en utilisant différentes concentration d'amoxicilline (64, 32, 16, 8, 4, 2 et 1µg/mL), une culture de chaque souche a été préparée dans 2mL de milieu de Mueller-Hinton assorti de 50% v/v d'eau lourde et de l'antibiotique. Un échantillon d'analyse témoin sans antibiotique a été constitué. Les échantillons d'analyse ont été ensuite incubés deux heures. Après incubation, les échantillons d'analyse ont été centrifugés et re-suspendus dans 30µL d'eau puis une goutte de 5µL est déposée sur une lame de fluorure de calcium (CaF₂, substance transparente aux infrarouges). La lame est séchée par exposition à une atmosphère chaude et sèche. Sur chaque échantillon d'analyse, 64 spectres sont acquis à l'aide d'un microscope FTIR à un grandissement de 15x, afin de s'abstraire de potentielles variations d'épaisseur ou de texture du dépôt.

On disposait ainsi de spectres d'analyse correspondant respectivement à une exposition des souches bactériennes à différentes concentrations en antibiotique. L'algorithme paramétré lors de la phase d'apprentissage a permis une mesure d'un taux de marquage de chaque échantillon, que l'on considère représentative de l'activité du métabolisme. La figure 4C présente, pour chaque concentration d'Amoxicilline, le taux de marquage. Sur la figure 4C, les résultats des caractérisations pour EC10 et EC207 ont été identifiés.

Si l'on constate une baisse du taux de marquage à forte concentration d'antibiotique, on en déduit que la bactérie y est sensible. Réciproquement, si le taux de marquage reste constant, même en présence d'une haute concentration en antibiotique, on en déduit que la bactérie y est résistante. Sur la figure 4C, on observe que la souche EC207 est résistante pour les concentrations d'antibiotiques mises en oeuvre, car le métabolisme se maintient à un niveau élevé pour l'ensemble des concentrations d'antibiotique. On observe également que la souche EC10 est sensible à l'antibiotique au-delà d'une certaine concentration, de l'ordre de 10 µg/mL. Sur la figure 4C, l'unité de l'axe des abscisses est la concentration d'antibiotique dans l'échantillon (µg/mL).

Un avantage du procédé est que la phase d'apprentissage peut être conduite en utilisant différentes souches bactériennes, avec le même marqueur métabolique, sachant qu'il est préférable que le milieu nutritif corresponde au milieu nutritif utilisé lors de la phase d'apprentissage. Cela améliore la robustesse de l'algorithme. Cela peut également permettre d'appliquer l'algorithme à des échantillons comportant des souches bactériennes n'ayant pas été utilisées lors de l'apprentissage.

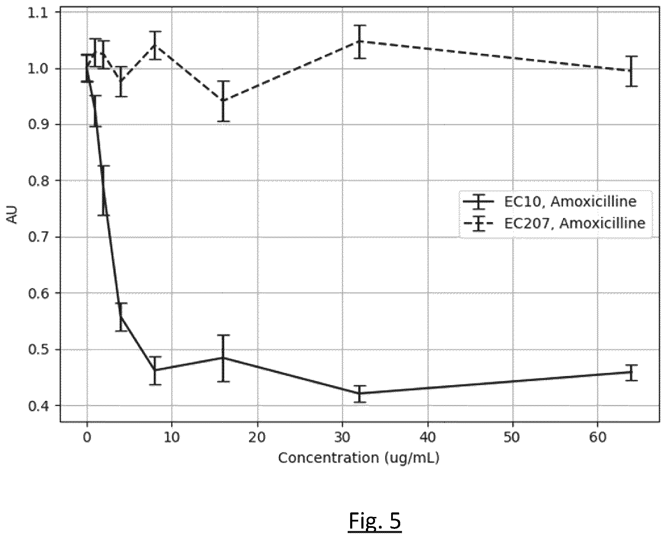

La figure 5 montre un exemple de détermination d'un métabolisme caractérisé en mettant en oeuvre l'algorithme de caractérisation tel que précédemment décrit, avec deux souches d'une bactérie *StaphilococcusSaprophyticus* (SS91, SS96) non utilisée lors de l'apprentissage. L'axe des abscisses correspond à la concentration d'antibiotique (Gentamicine). L'axe des ordonnées correspond à un taux de marquage. On observe que l'algorithme permet de déterminer, pour chaque souche, une concentration d'antibiotique au-delà de laquelle le taux de marquage diminue, ce qui traduit un ralentissement du métabolisme.

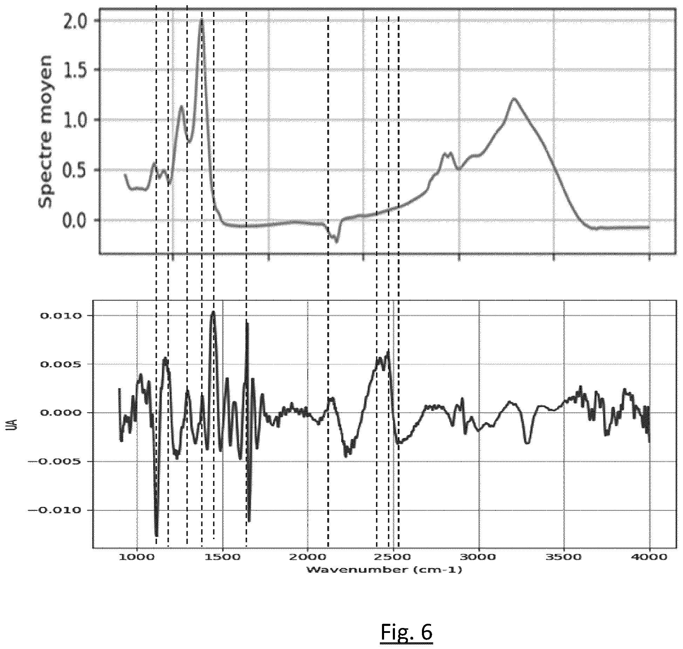

Dans l'exemple qui précède, on a décrit des spectres d'apprentissage et d'analyse s'étendant continûment entre 1200 cm⁻¹ et 4000 cm⁻¹. L'invention peut être mise en oeuvre avec certaines bandes spectrales seulement, par exemple des bandes spectrales d'intérêt, dans lesquels le classifieur permet une meilleure distinction entre les différentes classes. Sur la figure 6, on a représenté, par des pointillés, des bandes spectrales d'intérêt. Les courbes représentées sur la figure 6 correspondent à celles décrites en lien avec les figures 4A et 4B. On procède alors à une extraction du spectre en se limitant au contenu des bandes spectrales d'intérêt.

Bien que décrite ne lien avec une interaction d'une bactérie et d'un antibiotique, l'invention peut s'appliquer plus généralement à l'analyse d'interactions de bactéries avec des agents bactéricides ou bactériostatiques.

L'invention peut également s'appliquer à l'analyse d'interactions entre des champignons et un agent fongicide, ou entre d'autres types de microorganismes (microalgues, procaryotes) avec un agent biocide.

En outre, bien que décrite en lien avec une acquisition de spectres selon une modalité FTIR, l'invention s'applique à d'autres méthodes d'analyse spectrales, en transmission ou en réflexion, qu'elles soient non destructives ou destructives. Il peut en particulier s'agir de réflectance totale atténuée (Attenuated Total Reflectance), de spectrométrie Raman (pouvant être destructive) ou de spectrométrie de masse (destructive), par exemple selon une modalité MALDI-TOF. (Matrix Assisted Laser Desorption lonization - Time of Flight - spectrométrie de masse de type temps de vol avec source d'ionisation laser assistée par une matrice).

D'une façon générale, l'invention peut être mise en oeuvre avec une acquisition d'un spectre représentatif de la composition des microorganismes. Le marqueur métabolique est sélectionné de façon à être identifiable par le spectre.

## Revendications

1. Procédé de d'apprentissage d'un algorithme de caractérisation, configuré pour caractériser un métabolisme d'au moins une souche de microorganisme, le procédé comportant les étapes suivantes:
- a) la répartition de différents microorganismes de ladite souche dans différents échantillons d'apprentissage, les échantillons d'apprentissage comportant un milieu nutritif auquel a été ajouté un marqueur métabolique respectivement selon différentes concentrations, le marqueur métabolique étant destiné à être métabolisé par chaque microorganisme, au moins un échantillon étant tel qu'il ne comporte pas de marqueur métabolique, ou selon une concentration considérée comme négligeable ;
- b) l'acquisition de spectres d'apprentissage de microorganismes de chaque échantillon d'apprentissage ;
- c) à partir des spectres d'apprentissage acquis dans chaque échantillon d'apprentissage, le paramétrage de l'algorithme de caractérisation, de façon à caractériser, à partir de chaque spectre d'apprentissage, un métabolisme du microorganisme ;
le procédé étant **caractérisé en ce que** lors de l'étape b), l'acquisition des spectres d'apprentissage est effectuée en l'absence d'antibiotique ou d'antifongique dans chaque échantillon d'apprentissage.

2. Procédé selon la revendication 1, dans lequel :
- dans l'étape a), de microorganismes de différentes souches sont respectivement disposés dans différents échantillons d'apprentissage;
- l'étape b) comporte une acquisition de spectres d'apprentissage de microorganismes dans chaque échantillon d'apprentissage.

3. Procédé selon l'une quelconque des revendications précédentes, dans lequel l'algorithme de caractérisation est configuré pour classer le métabolisme dans une classe choisie parmi :
- une réduction du métabolisme ;;
- un métabolisme normal.

4. Procédé selon l'une quelconque des revendications précédentes, dans lequel dans l'étape a), les microorganismes sont disposés respectivement dans des milieux nutritifs identiques, les échantillons d'apprentissage différant les uns des autres par la concentration de marqueur métabolique ajoutée.

5. Procédé selon l'une quelconque des revendications précédentes, dans lequel le marqueur métabolique est un marqueur isotopique ou un marqueur chromogène.

6. Procédé selon l'une quelconque des revendications précédentes, dans lequel dans l'étape b), les microorganismes de chaque échantillon d'apprentissage sont interposés entre une source de lumière infrarouge et un photodétecteur, chaque spectre d'apprentissage étant un spectre d'absorption ou de transmission dans l'infrarouge.

7. Procédé selon l'une quelconque des revendications 1 à 6, dans lequel dans l'étape b), les microorganismes de chaque échantillon d'apprentissage sont disposés sur un support et exposées à un faisceau laser, chaque spectre d'apprentissage étant un spectre de masse.

8. Procédé de détermination d'une résistance d'un microorganisme à l'égard d'un agent antibiotique ou fongicide, le microorganisme étant disposé dans un échantillon d'analyse comportant le marqueur métabolique utilisé dans l'étape a) d'un procédé d'apprentissage selon l'une quelconque des revendications 1 à 7, le procédé comportant les étapes :
- i) mise en contact du microorganisme avec l'agent antibiotique ou fongicide ;
- ii) acquisition d'un spectre d'analyse du microorganisme, le spectre d'analyse étant du même type que les spectres d'apprentissage acquis dans chaque étape b) du procédé d'apprentissage ;
- iii) application d'un algorithme de caractérisation tel que défini dans l'étape c) du procédé d'apprentissage, au spectre résultant de l'étape ii), de façon à caractériser le métabolisme du microorganisme ;
- iv) détermination de la résistance du microorganisme à l'égard de l'agent antibiotique ou fongicide en fonction de la caractérisation effectuée lors de l'étape iii).

9. Procédé selon la revendication 8, dans lequel
- on dispose le microorganisme dans différents échantillons d'analyse, comportant respectivement différentes concentrations de l'antibiotique ;
- les étapes i) à iii) sont mises en oeuvre avec chaque échantillon d'analyse ;
- l'étape iv) comporte une détermination d'une concentration d'antibiotique ou de fongicide au-delà de laquelle le microorganisme n'est plus résistant.

10. Procédé selon l'une quelconque des revendications 8 ou 9, dans lequel
- le procédé d'apprentissage est mis en oeuvre avec des microorganismes d'apprentissage ;
- le procédé d'analyse est mis en oeuvre avec un microorganisme différent des microorganismes d'apprentissage.

11. Dispositif, comportant un spectromètre, configuré pour acquérir un spectre d'analyse d'un microorganisme, le dispositif comportant une unité de traitement programmée pour mettre en oeuvre un procédé selon la revendication 10.

12. Dispositif d'analyse selon la revendication 11, dans lequel le spectromètre est
- un spectromètre infra-rouge ;
- ou un spectromètre de masse ;
- ou un spectromètre Raman.
